# EUROPEAN PATENT SPECIFICATION

(11) **EP 2 289 875 B1**
(45) Date of publication and mention of the grant of the patent: **10.04.2013**
(21) Application number: 09802930.9
(22) Date of filing: 28.07.2009
(51) Int. Cl.: C07C 313/04

(54) **PROCESS FOR PRODUCING PERFLUOROALKANESULFINIC ACID SALT**
VERFAHREN ZUR HERSTELLUNG EINES PERFLUORALKANSULFINSÄURESALZES
PROCÉDÉ DE FABRICATION D'UN SEL D'ACIDE PERFLUOROALCANESULFINIQUE

(30) Priority: 30.07.2008 JP 2008195700; 27.07.2009 JP 2009174338
(43) Date of publication of application: 02.03.2011
(73) Proprietor: Central Glass Company, Limited, Yamaguchi 755-0001 (JP)
(72) Inventor: NANMYO, Tsutomu, Ube-shi, Yamaguchi 755-0001 (JP); KASHIWABA, Takashi, Ube-shi, Yamaguchi 755-0001 (JP); MORINAKA, Takayoshi, Ube-shi, Yamaguchi 755-0001 (JP); KAWAMOTO, Hiromi, Ube-shi, Yamaguchi 755-0001 (JP); INOUE, Susumu, Ube-shi, Yamaguchi 755-0001 (JP); KUME, Takashi, Ube-shi, Yamaguchi 755-0001 (JP)
(74) Representative: Manitz, Finsterwald & Partner GbR
(86) International application number: PCT/JP2009/063372
(87) International publication number: WO 2010/013687

(56) References cited:
- GB-A- 881 484
- JP-A- 48 056 619
- JP-A- 2001 316 353
- JP-A- 2005 247 782
- US-A- 3 919 301

## Description

### Technical Field

The present invention relates to a process for producing a perfluoroalkanesulfinate salt, which is useful as an intermediate for pharmaceutical and agrichemical raw materials.

### Background Art

As a conventional production process of a perfluoroalkanesulfinic acid derivative, Patent Document 1 and Non-Patent Document 1 each disclose a process of producing a perfluoroalkanesulfinic acid by reacting a perfluoroalkanesulfonyl fluoride with hydrazine to form a corresponding hydrazinium salt, and then, reacting the hydrazinium salt with an acid. (See [Chem. 1].)

Non-Patent Document 2 discloses a process of producing a sulfonic acid by reacting para-toluenesulfonyl chloride with a zinc metal powder to form a (para-toluenesulfinyl)zinc salt, reacting the salt with sodium carbonate to form a corresponding sulfinate, and then, treating the sulfinate with hydrochloric acid. (See [Chem. 2].)

Patent Document 2 discloses a process of producing a perhalomethanesulfinate salt by contacting a metal thiosulfate or a metal hydroxymethanesulfinate with a perhalomethane in a polar solvent. Patent Document 3 discloses a process of producing an alkali metal salt of a perfluoroalkanesulfinic acid by contacting a perfluoroalkanesulfonyl fluoride with an alkali metal salt of sulfurous acid in the presence of water. Non-Patent Document 3 discloses a process of producing potassium trifluoromethanesulfinate by reacting trifluoromethanesulfonyl chloride with potassium sulfite. Non-Patent Document 4 discloses a process of producing a sulfinate salt by reacting trifluoromethanesulfinic acid with sodium carbonate or potassium carbonate. (See [Chem. 3].)

Further, Non-Patent Document 5 discloses that the reaction of trifluoromethanesulphenyl chloride and sodium hydroxide gives sodium trifluoromethanesulfinate together with bis(trifluoromethyl)disulfide. (See [Chem. 4].)

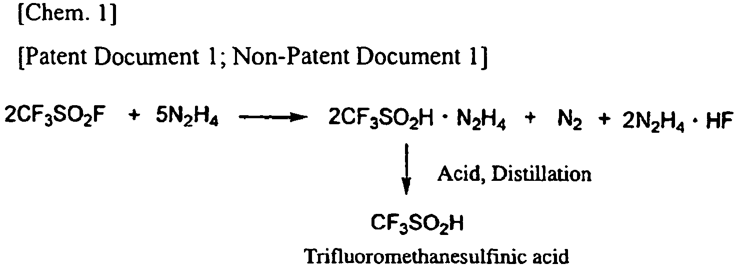

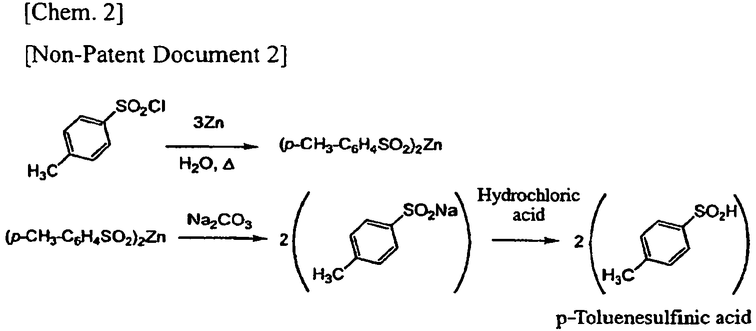

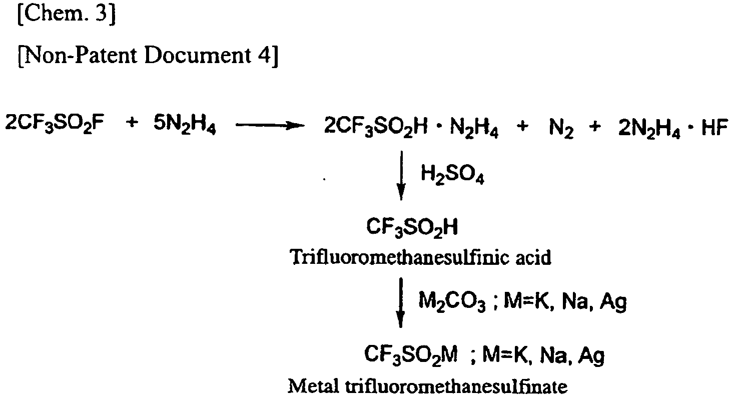

[Chem.4]
[Non-Patent Document 5]

3CF₃SCl + 4NaOH → CF₃SSCF₃ + CF₃SO₂Na + 3NaCl + 2H₂O

### Prior Art Documents

### Patent Documents

Patent Document 1:
   Japanese Laid-Open Patent Publication No. 48-56619 and US 3919301
Patent Document 2:
   Japanese Laid-Open Patent Publication No. 62-192351
Patent Document 3:
   Japanese Laid-Open Patent Publication No. 2001-316353

### Non-Patent Document

Non-Patent Document 1:
   Wechsberg, M. et al., Liebigs An. Chem., 33-39 (1973)
Non-Patent Document 2:
   Smith, M.B. et al., Organic Synthesis, Coll. Vol.2, 492 (1941)
Non-Patent Document 3:
   R.M. Scribner, J. Org. Chem., 31, 3671 (1966)
Non-Patent Document 4:
   H.W. Roesky and G. Holtschneider, J. Fluorine Chemistry, 7, 77 (1976)
Non-Patent Document 5:
   R.N. Haszeldine, J.M. Kidd, J. Chem. Soc., 2901-2910 (1955)

### Disclosure of the Invention

The processes of Patent Document 1 and of Non-Patent Document 4 appear at first glance to be favorable since the sulfinic acid or sulfinic acid metal salt can be produced with high yield. It is however necessary to purify the sulfinic acid by distillation, which may result in decomposition of the sulfinic acid. Further, there occurs hydrazine sulfate, which is a dangerous substance, during post-treatment operation. For these reasons, the processes of Patent Document 1 and Non-Patent Document 4 are somewhat difficult to apply for easy and safe industrial-scale production of the sulfinic acid or sulfinate salt.

The process of Patent Document 2 utilizes perhalomethane, which is relatively flame-retardant and easy to handle and has previously been used for a digestant etc. However, the production of perhalomethane is strictly restricted under the recent flon/halon regulations so that it is difficult to secure a stable supply of perhalomethane in the future. The process of Patent Document 2 is not desirable to apply not only because of the difficulty of stable perfluoromethane supply but also from the view point of environmental protection.

The processes of Patent Document 3 and Non-Patent Document 3 are somewhat difficult to apply industrially because of the difficulty of treating a liquid waste derived from the alkali metal sulfite after the reaction, the possibility of a low product yield (39%) and the need for a complicated purification operation.

The process of Non-Patent Document 2 has a danger of explosion in industrial-scale production due to the use of the zinc metal powder and provides a large amount of zinc waste. It is thus hardly said that the process of Non-Patent Document 2 is industrially applicable in point of cost performance.

It is accordingly an object of the present invention to provide a process for producing a perfluoroalkanesulfinate salt on an industrial scale with advantages in production cost, safety, convenience and facilitation.

The present inventors have made extensive researches to solve the above object and, as a result, have found an industrially advantageous process by which a perfluoroalkanesulfinate salt can be produced with high purity with the use of an industrially easily available perfluorialkanesulfonyl halide as a raw material. The present invention is based on this finding.

Namely, the present invention provides a process for producing a perfluoroalkanesulfinate salt as set forth by the following [Inventive Feature 1] to [Inventive Feature 11].

### [Inventive Feature 1]

A process for producing a perfluoroalkanesulfinate salt of the formula [1], comprising the following steps:
[Chem. 5]

**(R_{f}SO2)ₙM** [1]

where R_{f} represents a saturated or unsaturated C₁₋C₄ straight or branched perfluoroalkyl group; M represents an alkali metal or an alkaline-earth metal; and n represents an integer that is equal to the valency of the metal.

### [First Step]

The first step is a step of reacting a perfluoroalkanesulfonyl halide of the formula [2] with hydrazine to prepare a perfluoroalkanesulfinic acid hydrazinium salt (R_{f}SO₂HN₂H₄)
[Chem. 6]

**R_{f}SO₂X** [2]

where R_{f} is the same as defined in the formula [1]; and X represents fluorine, chlorine, bromine or iodine.

### [Second Step]

The second step is a step of forming a perfluoroalkanesulfinate salt by reaction of the perfluoroalkanesulfinic acid hydrazinium salt prepared in the first step with at least one base selected from the group consisting of alkali metal hydroxides, alkali metal carbonates, alkali metal hydrogencarbonates, alkaline-earth metal hydroxides, alkaline-earth metal carbonates and alkaline-earth metal hydrogencarbonates.

### [Third Step]

The third step is a step of adding an alkali metal halide or alkaline-earth metal halide (in which the alkali metal or alkaline-earth metal is the same as that of the base used in the second step) to the perfluoroalkanesulfinate salt formed in the second step and thereby obtaining the perfluoroalkanesulfinate salt as a crystalline precipitate.

### [Fourth Step]

The fourth step is a step of purifying the perfluoroalkanesulfinate salt obtained in the third step with the addition of at least one compound selected from the group consisting of alkali metal halides, alkaline-earth metal halides, alkali metal hydroxides, alkaline-earth metal hydroxides, alkali metal sulfates and alkaline-earth metal sulfates.

### [Inventive Feature 2]

A process for producing a potassium perfluoroalkanesulfinate of the formula [3], comprising the following steps:
[Chem. 7]

**R_{f}SO₂K** [3]

where R_{f} represents a saturated or unsaturated C₁-C₄ straight or branched perfluoroalkyl group.

### [First Step]

The first step is a step of reacting a perfluoroalkanesulfonyl fluoride of the formula [4] with hydrazine to prepare a perfluoroalkanesulfinic acid hydrazinium salt (R_{f}SO₂H·N₂H₄)
[Chem. 8]

**R_{f}SO₂F** [4]

where R_{f} is the same as defined in the formula [3].

### [Second Step]

The second step is a step of forming a potassium perfluoroalkanesulfinate by reaction of the perfluoroalkanesulfinic acid hydrazinium salt prepared in the first step with either potassium hydroxide, potassium carbonate or potassium hydrogencarbonate.

### [Third Step]

The third step is a step of adding potassium fluoride to the potassium perfluoroalkanesulfinate formed in the second step and thereby obtaining the potassium perfluoroalkanesulfinate as a crystalline precipitate.

### [Fourth Step]

The fourth step is a step of purifying the potassium perfluoroalkanesulfinate obtained in the third step with the addition of at least one compound selected from the group consisting of lithium chloride, sodium chloride, magnesium chloride, calcium chloride, lithium sulfate, sodium sulfate, magnesium sulfate, calcium sulfate and barium sulfate.

### [Inventive Feature 3]

The process according to Inventive Feature 1, wherein the first step is performed by charging the hydrazine in a reactor, and then, adding with stirring the perfluoroalkanesulfonyl halide at one time, sequentially or continuously, to the hydrazine in the reactor.

### [Inventive Feature 4]

The process according to any one of Inventive Features 1 to 3, wherein the first step is performed at a reaction temperature of -10 to 110°C.

### [Inventive Feature 5]

The process according to any one of Inventive Features 1 to 4, wherein the perfluoroalkanesulfinic acid hydrazinium salt prepared in the first step is used in the second step without purification.

### [Inventive Feature 6]

The process according to Inventive Feature 1 or 2, wherein, in the second step, the perfluoroalkanesulfinic acid hydrazinium salt is reacted with an equivalent molar amount of the base.

### [Inventive Feature 7]

The process according to Inventive Feature 1 or 2, wherein the perfluoroalkanesulfinate salt formed in the second step is used in the third step without purification.

### [Inventive Feature 8]

The process according to Inventive Feature 1 or 2, wherein the hydrazine present as a by-product in the third step is recovered and used as a reactant in the first step.

### [Inventive Feature 9]

The process according to Inventive Feature 1, wherein the alkali metal halide or alkaline-earth metal halide present in the reaction system is recovered and used as an additive in the third step.

### [Inventive Feature 10]

The process according to Inventive Feature 1, wherein an impurity present as a by-product including the at least one compound selected from the group consisting of alkali metal hydroxides, alkali metal carbonates, alkali metal hydrogencarbonates, alkaline-earth metal hydroxides, alkaline-earth metal carbonates and alkaline-earth metal hydrogencarbonates is recovered and mixed in a reaction solution before the purification of the fourth step.

### [Inventive Feature 11]

A process for producing perfluoroalkanesulfinic acid (CF₃SO₂H), comprising causing acid decomposition of the perfluoroalkanesulfinate salt produced by the process according to any one of Inventive Features 1 to 10.

In conventional processes, it is necessary to once form a trifluoromethanesulfinic acid hydrazinium salt, convert the trifluoromethanesulfinic acid hydrazinium salt to a sulfinic acid, and then, purify the sulfinic acid by distillation for production of the corresponding sulfinic acid metal salt. The processes of Patent Document 1 and Non-Patent Document 4 produce the sulfinic acid by reaction of the sulfonyl fluoride with hydrazine and acid decomposition of the resulting hydrazinium salt, but require distillation of the sulfinic acid and cause decomposition of the sulfinic acid during the distillation. It is thus somewhat difficult to apply the processes of Patent Document 1 and Non-Patent Document 4 for easy industrial-scale production of the sulfinic acid or sulfinic acid metal salt.

Further, it is known that the sulfinic acid is less stable than sulfonic acid and is thus likely to undergo a side reaction (disproportionation) (as reported in Non-Patent Document 2). It is also known that, in the process of Non-Patent Document 5, the presence of the strong base such as sodium hydroxide in the reaction system leads to decomposition of the trifluoromethanesulfinate salt to thereby produce the other compound (fluoroform (CHF₃)) as a by-product.

The process of Non-Patent Document 4 appears at first glance to be favorable for production of the potassium trifluoromethanesulfinate. However, in the case of actually performing this process while bearing in mind the application for industrial-scale production, there occurs decomposition of the sulfinic acid so that the product yield deteriorates to some extent. (See the after-mentioned reference example.) There also arises a difficulty of reaction control due to the distillation operation in the production of the sulfinic acid. It is thus somewhat undesirable to apply the process of Non-Patent Document 4 for industrial production of the potassium trifluoromethanesulfinate.

The present inventors have reached a finding that it is possible to produce a target perfluoroalkanesulfinate salt with high selectivity, without causing decomposition of the target product, by reacting a perfluoroalkanesulfonyl halide of the formula [2] with hydrazine to prepare a perfluoroalkanesulfinic acid hydrazinium salt (R_{f}SO₂H·N₂H₄) (the first step) and forming a perfluoroalkanesulfinic acid metal salt by reaction of the perfluoroalkanesulfinic acid hydrazinium salt prepared in the first step with at least one base selected from the group consisting of alkali metal hydroxides, alkali metal carbonates, alkali metal hydrogencarbonates, alkaline-earth metal hydroxides, alkaline-earth metal carbonates and alkaline-earth metal hydrogencarbonates (the second step). (See the following Scheme 1.) This process is suitable for industrial-scale production of the perfluoroalkanesulfinate salt, as compared to conventional processes, since there is no particular need to perform a distillation operation.

[Chem. 9]

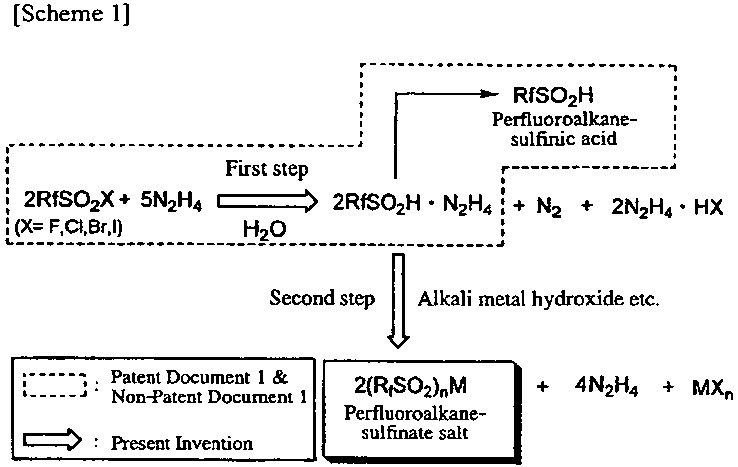

The perfluoroalkanesulfinate salt is easily soluble in water and is often obtained in the form of a reaction mixture thereof after the second step. As this sulfinate salt generally shows a very high solubility at ordinary temperatures, it has been very difficult to efficiently precipitate the sulfinate salt out of the reaction mixture.

The present inventors have also come up with a favorable finding that it is possible to obtain the perfluoroalkanesulfinate salt with high efficiency and high purity, without almost no decomposition, by precipitating the sulfinate salt efficiently with the addition of an alkali metal halide or alkaline-earth metal halide to the reaction mixture formed in the second step (the third step), and then, purifying the resulting crude sulfinate salt easily with the addition of at least one compound selected from the group consisting of alkali metal halides, alkaline-earth metal halides, alkali metal hydroxides, alkaline-earth metal hydroxides, alkali metal sulfates and alkaline-earth metal sulfates thereto (the fourth step). (See the following Scheme 2.)

[Chem. 10]

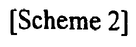

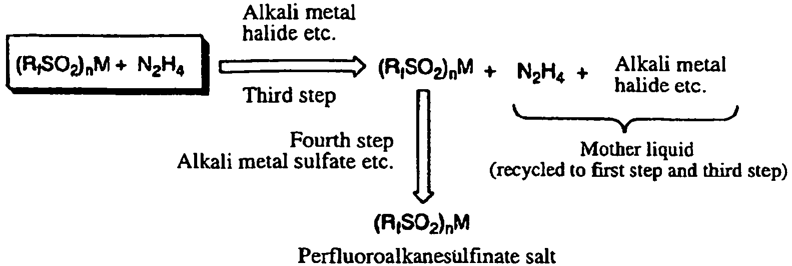

In general, it is preferable to purify the sulfinic acid metal salt to a high purity level through a route of acid decomposition, distillation and salt formation. However, this purification technique leads to increased number of process steps and often results in decomposition of the sulfinate salt. It has been found by the present inventors that the sulfinate salt can be easily obtained with high purity, without decomposition, by the above fourth step.

It has further been found that, after the addition of the alkali metal halide or alkaline-earth metal halide in the third step, the hydrazine or metal halide remaining in the mother liquid can be recovered and recycled as the reactants in the first and third steps. This enables substantial reduction in wastes.

The sulfinate salt obtained in the fourth step can be easily converted to a perfluoroalkanesulfinic acid (R_{f}SO₂H) by acid decomposition.

In this way, the present invention provides a very useful process for easy and safe industrial production of the perfluoroalkanesulfinate salt through the above-mentioned first to fourth steps.

### Detailed Description

According to the present invention, a perfluoroalkanesulfinate salt, which is useful as an intermediate for pharmaceutical and agrichemical raw materials, can be easily produced with high selectivity and yield and at low cost.

The present invention will be described in detail below. The present invention provides a process for producing a perfluoroalkanesulfinate salt of the formula [1], including the following steps of: reacting a perfluoroalkanesulfonyl halide of the formula [2] with hydrazine to prepare a perfluoroalkanesulfinic acid hydrazinium salt (R_{f}SO₂H·N₂H₄) (the first step); forming a perfluoroalkanesulfinic acid metal salt by reaction of the perfluoroalkanesulfinic acid hydrazinium salt prepared in the first step with at least one base selected from the group consisting of alkali metal hydroxides, alkali metal carbonates, alkali metal hydrogencarbonates, alkaline-earth metal hydroxides, alkaline-earth metal carbonates and alkaline-earth metal hydrogencarbonates (the second step); adding an alkali metal halide or alkaline-earth metal halide to the perfluoroalkanesulfinate salt formed in the second step and thereby obtaining the metal salt as a crystalline precipitate (the third step); and purifying the metal salt obtained in the third step with the addition of at least one compound selected from the group consisting of alkali metal halides, alkaline-earth metal halides, alkali metal hydroxides, alkaline-earth metal hydroxides, alkali metal sulfates and alkaline-earth metal sulfates (the fourth step). In the present invention, the "alkaline-earth metal" refers to Be, Mg, Ca, Sr, Ba or Ra.

The production process of the present invention is herein summarized in the following Scheme 3.

[Chem. 11]

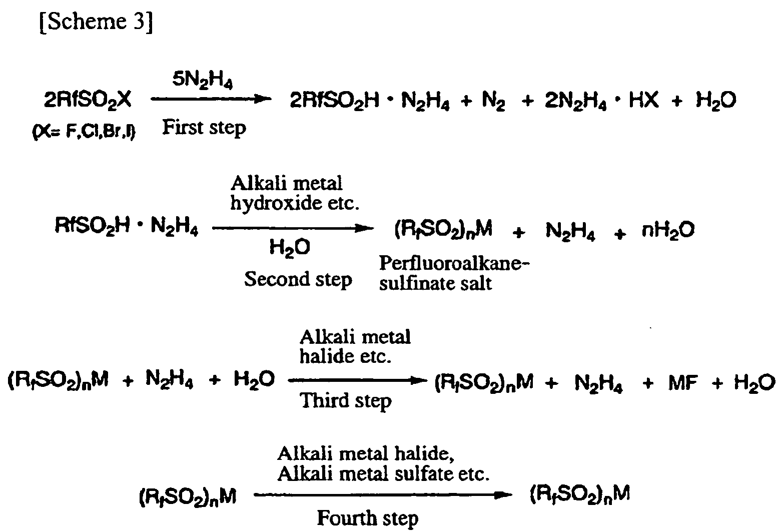

The perfluoroalkanesulfonyl halide, which is the starting material of the first step, can be produced by a conventionally known method. For example, trifluoromethanesulfonyl fluoride can be produced by a method disclosed in U.S. Patent No. 2,732,398, i.e., by electrolytic fluorination of trifluoromethanesulfonyl chloride in an anhydrous hydrogen fluoride. Alternatively, trifluoromethanesulfonyl fluoride can produced by a method indicated in the following Scheme 4, i.e., by fluorinating methanesulfonyl chloride with sodium fluoride and then subjecting the resulting methanesulfonyl fluoride to electrolytic fluorination in an anhydrous hydrogen fluoride.

Further, trifluoromethanesulfonyl chloride can be produced by a method disclosed in Japanese Laid-Open Patent Publication No. 2000-191634 or Japanese Laid-Open Patent Publication No. 2001-191635.

[Chem. 12]

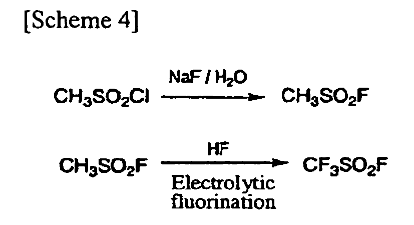

The first step will be first explained below. In the first step, the perfluoroalkanesulfinic acid hydrazinium salt (R_{f}SO₂H·N₂H₄) is prepared by reaction of the perfluoroalkanesulfonyl halide of the formula [2] with hydrazine. As the perfluoroalkanesulfonyl halide, there are generally used those having a saturated or unsaturated straight or branched perfluoroalkyl group with a carbon number of 1 to 4, preferably a carbon number of 1 to 3, particularly preferably a carbon number of 1 (i.e. trifluoromethyl). Specific examples of the perfluoroalkanesulfonyl halide are trifluoromethanesulfonyl fluoride, pentafluoroethanesulfonyl fluoride, heptafluoropropanesulfonyl fluoride, nonafluorobutanesulfonyl fluoride, trifluoromethanesulfonyl chloride, pentafluoethanesulfonyl chloride, heptafluoropropanesulfonyl chloride, nonafluorobutanesulfonyl chloride, trifluoromethanesulfonyl bromide, pentafluoroethanesulfonyl bromide, heptafluoropropanesulfonyl bromide, nonafluorobutanesulfonyl bromide, trifluoromethanesulfonyl iodide, pentafluoroethanesulfonyl iodide, heptafluoropropanesulfonyl iodide and nonafluorobutanesulfonyl iodide. Among others, trifluoromethanesulfonyl fluoride, pentafluoroethanesulfonyl fluoride, heptafluoropropanesulfonyl fluoride, trifluoromethanesulfonyl chloride, pentafluoroethanesulfonyl chloride, heptafluoropropanesulfonyl chloride, trifluoromethanesulfonyl bromide, pentafluoroethanesulfonyl bromide, heptafluoropropanesulfonyl bromide, trifluoromethanesulfonyl iodide, pentafluoroethanesulfonyl iodide and heptafluoropropanesulfonyl iodide are preferred. Particularly preferred are trifluoromethanesulfonyl fluoride, pentafluoroethanesulfonyl fluoride, trifluoromethanesulfonyl chloride, pentafluoroethanesulfonyl chloride, trifluoromethanesulfonyl bromide, pentafluoroethanesulfonyl bromide, trifluoromethanesulfonyl iodide and pentafluoroethanesulfonyl iodide.

It is stoichiometrically necessary to use the hydrazine in an amount of 2.5 mol per 1 mol of the perfluoroalkanesulfonyl halide in the first step. The amount of the hydrazine used is generally in the range of 0.5 to 10 mol and is preferably set as appropriate in the range of 2.5 to 5 mol.

When the amount of the hydrazine is less than 2.5 mol, it may cause a deterioration in the reaction yield. When the amount of the hydrazine exceeds 10 mol, there is no problem in the progress of the reaction but also no particular merit in terms of the reaction rate, yield and economy.

In the first step, the hydrazine can be used in the form of either a simple substance (N₂H₄) or a hydrate (e.g. hydrazine monohydrate (N₂H₄·H₂O)).

Further, the reaction can be performed under the coexistence of a solvent in the reaction system in the first step. Herein, the solvent refers to an inert solvent that is not directly involved in the reaction. Specific examples of the solvent are: aliphatic hydrocarbon solvents such as n-hexane, cyclohexane and n-heptane; aromatic hydrocarbon solvents such as benzene, toluene and xylene; halogenated hydrocarbon solvents such as methylene chloride, chloroform and 1,2-dichloroethane; ether solvents such as diethyl ether, tetrahydrofuran and tert-butyl methyl ether; nitrile solvents such as acetonitrile and propionitrile; alcohol solvents such as methanol, ethanol, isopropanol, n-butanol and phenol; dimethyl sulfoxide; and water.

Among others, halogenated hydrocarbon solvents such as methylene chloride, chloroform and 1,2-dichloroethane, ether solvents such as diethyl ether, tetrahydrofuran and tert-butyl methyl ether, alcohol solvents such as methanol, ethanol, isopropanol, n-butanol and phenol and water are preferred. More preferred are alcohol solvents and water. These solvents can be used solely or in combination thereof.

The amount of the solvent used is set as appropriate in the range of generally 0.5 to 10 mol, preferably 1 to 5 mol, per 1 mol of the perfluoroalkanesulfonyl halide.

As the hydrazine is in liquid form at ordinary temperatures and pressures, the amount of the solvent largely varies depending on the amount of the hydrazine used. The amount of the solvent can be thus adjusted as appropriate by a person skilled in the art within the above-specified range depending on the amount of the hydrazine in such a manner that the reaction proceeds efficiently in the first step.

For example, water can be used as the solvent in an amount that the resulting aqueous hydrazine solution has a concentration of generally 10 to 64 mass%, preferably 10 to 40 mass%, more preferably 15 to 35 mass%. It is one preferred embodiment of the present invention to add water in such a manner that the concentration of the aqueous hydrazine solution ranges from 20 to 30 mass%.

The perfluoroalkanesulfonyl halide is present as a liquid or gas at ordinary temperatures and pressures depending on the kind thereof. There is no particular limitation on the form of the perfluoroalkanesulfonyl halide during the material charging operation. The perfluoroalkanesulfonyl halide can be in either liquid form or gas form during the material charging operation. The form of the perfluoroalkanesulfonyl halide during the material charging operation can be selected as appropriate by a person skilled in the art.

The pressure is generally 0.1 to 10 MPa, preferably 0.1 to 5 MPa, more preferably 0.1 to 2 MPa, in the first step.

Further, the reaction temperature is generally -10 to 110°C, preferably 25 to 80°C, more preferably 40 to 70°C, in the first step.

There is no particular limitation on the method of charging the perfloroalkanesulfonyl halide for reaction with the hydrazine. In general, the perfluoroalkanesulfonyl halide can be added to a reactor after charging the hydrazine into the reactor. In the case of using trifluoromethanesulfonyl fluoride as the perfluoroalkanesulfonyl halide, it is one preferred embodiment of the present invention to charge the hydrazine in the reactor, and then, adding with stirring or pump circulation the fluoride at one time, sequentially or continuously as in the after-mentioned examples.

In the first step, nitrogen (N₂) gas appears with the progress of the reaction. It is thus conceivable to open one side of the reactor and thereby discharge the nitrogen gas from the reaction zone.

As the reactor, there can be used any of those made of metal materials such as stainless steel, Hastelloy and Monel in the case of performing the reaction under pressurized conditions. In the case of performing the reaction under ordinary pressure conditions, the reactor can be selected as appropriate by a person skilled in the art.

There is no particular limitation on the material of the reactor as long as it is capable of resisting and withstanding the pressure in the case of performing the reaction under ordinary pressure conditions or pressurized conditions. The reactor may be formed with an inner lining of tetrafluoroethylene resin, chlorotrifluoroethylene resin, vinylidene fluoride resin, PFA resin, polypropylene resin, polyethylene resin, or glass etc. Further, a glass container can be used as the reactor.

In the case where the perfloroalkanesulfonyl halide is in gas form, it is preferable to keep the reactor at low temperatures or use a low-temperature condenser in the reactor in order to prevent the perfloroalkanesulfonyl halide from being released from the reaction zone while introducing the perfloroalkanesulfonyl halide into the reaction system. Further, it is effective to utilize any contact efficiency improvement means such as gas introduction rate control, stirring equipment, gas blowing equipment or sparger (porous diffusion tube) as appropriate in the case of using the ordinary reactor. The use of a scrubber-type reactor with a pump circulation mechanism is a particularly preferred embodiment for improvement in contact efficiency.

There is no particular limitation on the reaction time. The reaction time is generally set to 24 hours or less. It is preferable to determine, as the end of the reaction, the time at which the raw substrate materials have almost disappeared while monitoring the progress of the reaction by any analytical means such as gas chromatography, IR, ion chromatography or NMR.

There is also no particular limitation on the treatment operation after the reaction. The reactant remaining after the completion of the reaction can be treated by any ordinary organic synthesis treatment technique.

It has been found that it is possible to use the perfluoroalkanesulfinic acid hydrazinium salt prepared in the first step, without purification, as the starting material of the second step so that the reaction can proceed favorably in the second step with no particular loss of yield. In the reaction system, hydrazine hydrohalide (N₂H₄·HX) is generated simultaneously with the perfluoroalkanesulfinic acid hydrazinium salt (R_{f}SO₂H·N₂H₄). The perfluoroalkanesulfinic acid hydrazinium salt is thus obtained in the form of a reaction mixture thereof in the first step. It is a preferred embodiment to use this reaction mixture as it is, without purification, as the raw material of the second step in terms of the productivity.

Next, the second step will be explained below. In the second step, the perfluoroalkanesulfinate salt is formed by reaction of the perfluoroalkanesulfinic acid hydrazinium salt prepared in the first step with at least one base selected from the group consisting of alkali metal hydroxides, alkali metal carbonates, alkali metal hydrogencarbonates, alkaline-earth metal hydroxides, alkaline-earth metal carbonates and alkaline-earth metal hydrogencarbonates.

Among the above kinds of bases usable in the second step, the alkali metal hydroxides includes lithium hydroxide (LiOH), potassium hydroxide (KOH), rubidium hydroxide (RbOH) and cesium hydroxide (CsOH); the alkali metal carbonates includes lithium carbonate (Li₂CO₃), potassium carbonate (K₂CO₃), sodium carbonate (Na₂CO₃), rubidium carbonate (Rb₂CO₃) and cesium carbonate (Cs₂CO₃); the alkali metal hydrogencarbonates includes lithium hydrogencarbonate (LiHCO₃), potassium hydrogencarbonate (KHCO₃), sodium hydrogencarbonate (NaHCO₃), rubidium hydrogencarbonate (RbHCO₃) and cesium hydrogencarbonate (CsHCO₃); the alkaline-earth metal hydroxides includes magnesium hydroxide (Mg(OH)₂), calcium hydroxide (Ca(OH)₂), barium hydroxide (Ba(OH)₂) and strontium hydroxide (Sr(OH)₂); the alkaline-earth metal carbonates includes magnesium carbonate (MgCO₃), calcium carbonate (CaCO₃), barium carbonate (BaCO₃) and strontium carbonate (SrCO₃); and the alkaline-earth metal hydrogencarbonates includes barium hydrogencarbonate (Ba(HCO₃)₂), magnesium hydrogencarbonate (Mg(HCO₃)₂), calcium hydrogencarbonate (Ca(HCO₃)₂) and strontium hydrogencarbonate (Sr(HCO₃)₂). These bases can be used solely or in combination of two or more thereof. In the case of using two or more kinds of bases in combination, it is preferable that these bases have the same alkali metal or alkaline-earth metal.

The amount of the base used in the second step is 2 mol or more, preferably 5 mol or less, more preferably 3 mol or less, per 1 mol of the perfluoroalkanesulfinic acid hydrazinium salt. The reaction proceeds even when the amount of the base exceeds 5 mol i.e. when the base is used excessively in the reaction. However, the use of excessive base may cause a deterioration in the yield of the reaction by decomposition of the perfluoroalkanesulfinic acid hydrazinium salt or perfluoroalkanesulfinate salt. Further, the conversion rate of the reaction may deteriorate when the amount of the base is less than 2 mol.

It has also been found that it is possible to improve the selectivity of the target compound, with almost no by-product formation, by reacting the perfluoroalkanesulfinic acid hydrazinium salt with the equivalent molar amount of the base i.e. by using 2 mol of the base per 1 mol of the hydrazinium salt.

In the second step, a solvent can be used together with the base. In the case of using water as the solvent, it is preferable to use the water in an amount that the concentration of the base in the resulting aqueous solution is in the range of generally 10 to 70 mass%, preferably 20 to 60 mass%, more preferably 30 to 60 mass%. The addition of too less water makes it difficult to stir the reaction system. The addition of too much water provides no industrial and economical merit due to the need for a complicated treatment operation after the reaction and to the need for a larger than usual reactor.

It is one preferred embodiment to use the aqueous potassium hydroxide solution with a concentration of 48 mass%.

There can be used an organic solvent other than the water. Examples of the organic solvent are water-soluble solvents such as ethers including diethyl ether, dioxane, tetrahydrofuran and ethylene glycol dimethyl ether. Further, the organic solvent can be used in combination with the water. The amount of the solvent used relative to the perfluoroalkanesulfinic acid hydrazinium salt is set as appropriate in the range of generally 0.5 to 10 times in volume, preferably 1 to 7 times in volume. There is however less merit in using the organic solvent other than water as the reaction proceeds sufficiently with the use of water.

There is no particular limitation on the reaction temperature in the second step. The reaction temperature is generally -10 to 110°C, preferably 25 to 80°C. When the reaction temperature is lower than -10°C, the reaction does not proceed sufficiently. This becomes a cause of yield deterioration and may raise a problem that it takes a long time to complete the reaction due to a decrease of reaction speed. On the other hand, it becomes likely that a by-product will occur when the reaction temperature is higher than 110°C.

There is no particular limitation on the reaction time. The reaction time is generally set to 24 hours or less. It is preferable to determine, as the end of the reaction, the time at which the raw substrate materials have almost disappeared while monitoring the progress of the reaction by any analytical means such as ion chromatography or NMR.

There can be used any reactor by which the reaction proceeds sufficiently at ordinary temperatures and pressures in the second step. The reactor can be selected from those made of metal materials, such as stainless steel, Hastelloy and Monel, with or without an inner lining of tetrafluoroethylene resin, chlorotrifluoroethylene resin, vinylidene fluoride resin, PFA resin, polypropylene resin, polyethylene resin, or glass etc.

After the reaction of the second step, the resultant perfluoroalkanesulfinic acid metal salt is often being dissolved in the solvent in the reaction system because of its very high solubility. Further, a large amount of hydrazine by-product derived from the starting material is contained in the reaction system. The sulfinic acid metal salt is thus obtained in the form of a reaction mixture containing the solvent.

It has favorably been found that it is possible to use the reaction mixture obtained in the second step, without purification, as the starting material of the third step so that the sulfinate salt can be precipitated in crystalline form in the third step with no particular loss of yield.

It is conceivable that the reaction solution immediately after the addition of the alkali metal hydroxide etc. in the second step can be used, as it is without purification, as the starting material of the after-mentioned fourth step. In the present invention, however, when the third step is bypassed, the target compound of the fourth step, i.e., perfluoroalkanesulfinate salt may deteriorate in purity due to the coexistence of perfluoroalkanesulfonic acid salt etc. In order to obtain the perfluoroalkanesulfinic acid metal salt with high purity, it is preferable to go through the third step in the present invention.

It has also been found that the hydrazine present as the by-product after the second step can be recovered at the stage of post-treatment operation of the after-mentioned third step and recycled as the reactant in the above-mentioned first step.

Further, it has been industrially advantageously found that the recovery amount of the hydrazine can be increased by recovering the hydrazine at the stage of post-treatment operation of the third step rather than at the stage of post-treatment operation of the second step. (See the after-mentioned reference example.)

For example, it is a particularly preferred embodiment of the present invention to use the solvent-containing reaction mixture obtained in the second step, as the starting material of the third step without separation operation such as purification, and to recycle the by-product (hydrazine) derived from the second step by the post-treatment of the reaction solution after the third step for much higher productivity than conventional processes and for substantial reduction in wastes. (See the following Scheme 5.)

[Chem. 13]

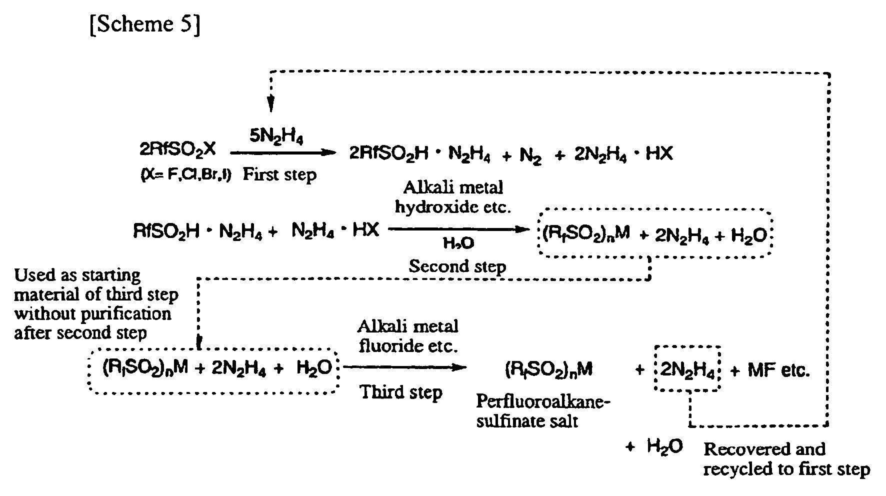

The third step will be next explained below. In the third step, the perfluoroalkanesulfinate salt formed in the second step is precipitated in crystalline form with the addition of alkali metal halide or alkaline-earth metal halide.

As mentioned above, the perfluoroalkanesulfinate salt is obtained in the form of the solvent-containing reaction mixture in the second step. In this case, the solubility of the perfluoroalkanesulfinate salt in the solvent becomes lowered under the salting-out effect with the addition of the alkali metal halide or alkaline-earth metal halide to the reaction mixture so that the sulfinate salt can be easily precipitated by concentration etc. Further, the perfluoroalkanesulfinate salt can be efficiently obtained as the crystalline precipitate by solvent concentration.

Specific examples of the alkali metal halide usable in the third step are lithium fluoride (LiF), sodium fluoride (NaF), potassium fluoride (KF), rubidium fluoride (RbF), cesium fluoride (CsF), lithium chloride, sodium chloride, potassium chloride, rubidium chloride, cesium chloride, lithium bromide, sodium bromide, potassium bromide, rubidium bromide, cesium bromide, lithium iodide, sodium iodide, potassium iodide, rubidium iodide and cesium iodide. Specific examples of the alkaline-earth metal halide usable in the third step are magnesium fluoride, calcium fluoride, strontium fluoride, barium fluoride, magnesium chloride, calcium chloride, strontium chloride, barium chloride, magnesium bromide, calcium bromide, strontium bromide, barium bromide, magnesium iodide, calcium iodide, strontium iodide and barium iodide.

Preferably, the metal of the alkali metal halide or alkaline-earth metal halide used in the third step is of the same kind as that of the base used in the above-mentioned second step. When the metal halide used in the third step is of different metal from that of the sulfinate salt obtained after the second step, it is difficult to sufficiently precipitate and purify the perfluoroalkanesulfinate salt in the third and fourth steps. The perfluoroalkanesulfinate salt can be easily precipitated in crystalline form when the alkali metal halide or alkaline-earth metal halide used in the third step is of the same metal as that of the salt used in the second step.

There is no particular limitation on the form of the alkali metal halide or alkaline-earth metal halide added in the third step. This halide is present as a solid at ordinary temperatures and pressures. In the third step, the halide can be added as it is or may be added into a solvent and thereby reacted in the form of a solution.

The amount of the alkali metal halide or alkaline-earth metal halide used is generally 1 to 10 mol, preferably 2 to 6 mol, more preferably 3 to 4 mol, per 1 mol of the perfluoroalkanesulfinate salt. The yield of the reaction may deteriorate when the amount of the alkali metal halide or alkaline-earth metal halide is less than 1 mol. When the amount of the alkali metal halide or alkaline-earth metal halide exceeds 10 mol, there is no problem in crystalline precipitation. However, the addition of more than the necessary amount of the alkali metal halide or the like makes it likely that there will arises a problem of deterioration in the productivity of the purification operation in the after-mentioned fourth step.

In the case where the alkali metal halide or alkaline-earth metal halide is dissolved in the solvent and reacted in solution form, the amount of the solvent used can be adjusted as appropriate by a person skilled in the art depending on the amount of the alkali metal halide or alkaline-earth metal halide.

For example, it is preferable to use water as the solvent in an amount that the concentration of the alkali metal halide or alkaline-earth metal halide in the resulting aqueous solution is in the range of generally 10 to 50 mass%, preferably 20 to 45 mass%, more preferably 30 to 40 mass%. It is one preferred embodiment to use the aqueous halide solution with a concentration of 10 to 20 mass%.

In the case where water is contained in the starting material of the third step, the amount of the water used at the time of addition of the alkali metal halide or alkaline-earth metal halide can be adjusted as appropriate by a person skilled in the art to within the above aqueous solution concentration range in view of the amount of the water contained in the starting material.

There is no particular limitation on the reaction time in the third step. As the target perfluoroalkanesulfinate salt is gradually precipitated out during this step, it is preferable to determine, as the end of the reaction, the time at which the precipitation of the salt has almost finished while monitoring the progress of the salt precipitation.

In the third step, the temperature can be set as appropriate depending on the boiling and freezing points of the solvent used. Generally, the crystalline precipitate of the perfluoroalkanesulfinate salt is obtained in the temperature range of -10 to 100°C, preferably -10 to 80°C, more preferably 0 to 60°C.

There is no particular limitation on the reactor used in the third step. The reactor can be selected from those made of metal materials, such as stainless steel, Hastelloy and Monel, with or without an inner lining of tetrafluoroethylene resin, chlorotrifluoroethylene resin, vinylidene fluoride resin, PFA resin, polypropylene resin, polyethylene resin, or glass etc.

The post-treatment operation of the third step can be performed by any ordinary organic synthesis treatment technique. For example, it is conceivable to obtain the perfluoroalkanesulfinate salt in crystalline form by partially concentrating under reduced pressure the reaction solution in which the solid precipitate is contained, separating the precipitate from the mother liquid by filtration etc. and drying the precipitate.

In the third step, there is a case that the hydrazine by-product derived from the second step remains in the reaction system. The hydrazine generated as the by-product coexists with the perfluoroalkanesulfinate salt after the second step. The reaction system thus contains a larger amount of hydrazine generated as the by-product when used as the starting raw material of the third step without purification as in the after-mentioned examples than when used with purification.

It has been industrially favorably found that it is possible to easily recover and use the hydrazine as the reactant in the first step by partially concentrating the reaction solution containing the hydrazine by-product. More specifically, when the reaction solution containing the precipitated solid is partially concentrated under reduced pressure, the hydrazine generated as the by-product and remaining in the reaction system after the second step is extracted into the distillate. The resulting hydrazine distillate is recovered and recycled as the reactant in the first step. This is a preferred embodiment of the present invention. (See the following Scheme 6.)

[Chem. 14]

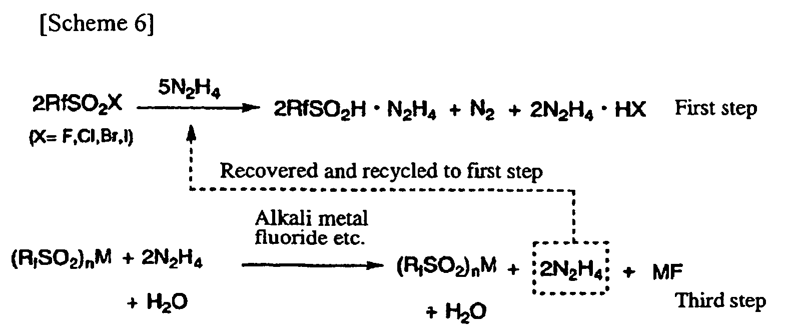

There is also a case that the hydrazine remains, together with the perfluoroalkanesulfinate salt, in the reaction solution after the distillation. In this case, the hydrazine can be easily separated from the target perfluoroalkanesulfinate salt compound by concentration and filtration as shown in the following Scheme 7.

The third step, in which the perfluoroalkanesulfinate salt is precipitated in crystalline form, involves concentration and filtration after the crystalline precipitation as shown in the following Scheme 7. The alkali metal halide or alkaline-earth metal halide is thus contained in the resulting filtrate. Further, an impurity such as a slight amount of by-product e.g. hydrazine, a salt of perfluoroalkanesulfinic acid (R_{f}SO₂H) and a salt of perfluoroalkanesulfonic acid (R_{f}SO₃H) is often contained in the filtrate.

The reaction solution immediately before the addition of the alkali metal halide or alkaline-earth metal halide in the third step can be used as it is as the starting raw material of the subsequent fourth step. However, the use of this reaction solution without precipitation and separation can lead to a deterioration in the purity of the target alkali metal salt compound of the fourth step due to the coexistence of the perfluoroalkanesulfonic acid salt. It is thus preferable to perform the above precipitation and separation operations of the third step in order to obtain the sulfinate salt with high purity.

Furthermore, it has been favorably found that it is possible to recover and recycle the alkali metal halide or alkaline-metal halide present in the reaction system as the additive in the third step. More specifically, after the precipitation of the perfluoroalkanesulfinate salt, the alkali metal halide or alkaline-earth metal halide contained in the filtrate can be recovered and recycled as the additive in the third step.
For example, it is a particularly preferred embodiment to, after subjecting potassium perfluoroalkanesulfinate to solvent distillation, filter out the solid, add an oxidizer such as 30% hydrogen peroxide to the filtrate for oxidation treatment of the perfluoroalkanesulfinic acid (R_{f}SO₂H + 30% H₂O₂ → R_{f}SO₃H) together with the hydrazine, pass the treated solution through a tower packed with an ion-exchange resin (e.g. available under the trade name of "Amberlyst" from Organo Tokyo Corporation) and thereby separate and recover the perfluoroalkanesulfonic acid alkali metal salt, recover the potassium fluoride from the filtrate, and then, recycle the potassium fluoride as the additive in the third step. (See the following Scheme 7.)

[Chem. 15]

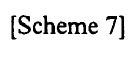

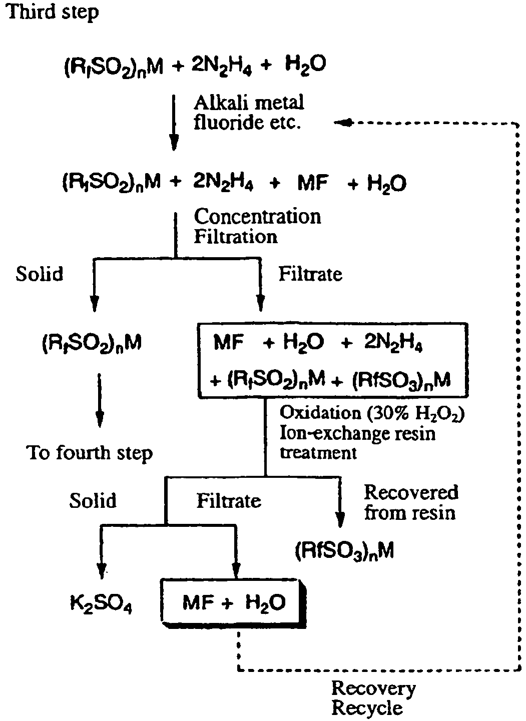

As mentioned above, the alkali metal fluoride or alkaline-earth metal fluoride is contained in the solution that has been subjected to the oxidation treatment and passed through the ion-exchange resin packed tower after the precipitation of the third step. The alkali metal fluoride can be recovered by separately adding an alkali metal hydroxide (sodium hydroxide etc.) to the above aqueous solution. For example, when an aqueous solution of sodium hydroxide is added to the impurity containing potassium fluoride, the potassium fluoride is converted to less-soluble sodium fluoride. The sodium fluoride can be thus isolated. The isolated sodium fluoride can be recycled as the reactant for preparation of the trifluoromethanesulfonyl fluoride used as the raw material of the first step.

The fourth step will be next explained below. In the fourth step, the perfluoroalkanesulfinate salt obtained in the third step is purified with the addition of at least one compound selected from the group consisting of alkali metal halides, alkaline-earth metal halides, alkali metal hydroxides, alkaline-earth metal hydroxides, alkali metal sulfates and alkaline-earth metal sulfates.

The alkali metal fluoride and alkali metal sulfate etc. are often contained as the impurity in the crystalline solid of the perfluoroalkanesulfinate salt obtained in the third step. In the fourth step, at least one metal salt selected from the group consisting of alkali metal halides, alkaline-earth metal halides, alkali metal hydroxides, alkaline-earth metal hydroxides, alkali metal sulfates and alkaline-earth metal sulfates is added to the perfluoroalkanesulfinate salt to thereby efficiently remove such an impurity and obtain the target compound with high purity. (See the following Scheme 8.)

Among others, the metal salt is preferably at least one selected from the group consisting of lithium chloride, sodium chloride, magnesium chloride, calcium chloride, lithium sulfate, sodium sulfate, magnesium sulfate, calcium sulfate and barium sulfate.

For example, less soluble fluoride (sodium fluoride) and potassium sulfate are generated by the addition of an aqueous sodium sulfate solution to the potassium trifluoromethanesulfinate containing therein potassium fluoride. Because of the difference between the solubility of the potassium trifluoromethanesulfinate and the solubility of the sodium fluoride and potassium sulfate, it becomes easier to separate the potassium sulfinate and thus possible to obtain the potassium sulfinate with high purity. This is a particularly preferred embodiment of the present invention. (See Scheme 9 as follows.)

[Chem. 16]

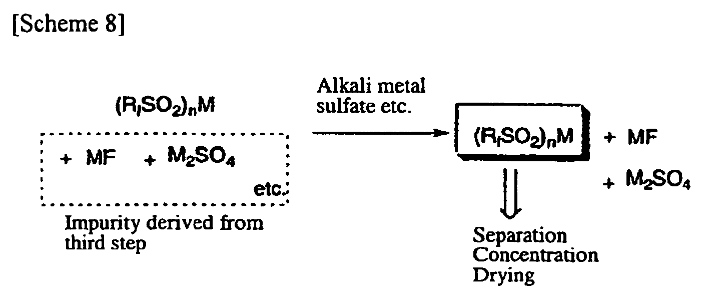

[Chem. 17]

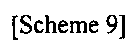

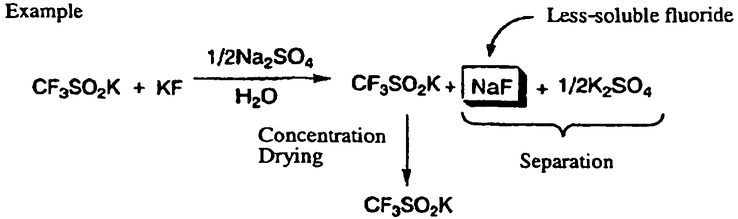

The amount of the at least one compound selected from the group consisting of alkali metal halides, alkaline-earth metal halides, alkali metal hydroxides, alkaline-earth metal hydroxides, alkali metal sulfates and alkaline-earth metal sulfates is generally 0.5 to 2.5 mol, preferably 0.5 to 1.0 mol, per 1 mol of the alkali metal halide or alkaline-earth metal halide contained in the perfluoroalkanesulfinate salt used as the starting material of the fourth step.

In the case of using, among the above compounds, the alkali metal hydroxide or alkaline-earth metal hydroxide in the fourth step, metal hydroxide is generated in the reaction system as a by-product by the reaction of the hydroxide with the alkali metal fluoride.

The target compound of the fourth step, i.e., perfluoroalkanesulfinate salt could be decomposed by the metal hydroxide. It is thus desirable to treat the generated metal hydroxide with an acid (such as sulfuric acid or hydrochloric acid). It is particularly desirable to use the equivalent molar amount of the acid per 1 equivalent weight of the metal hydroxide as the treatment of the metal hydroxide with more than the equivalent amount of the acid may result in decomposition of the target perfluoroalkanesulfinate salt.

In the fourth step, the reaction temperature can be set as appropriate depending on the kind of the additive. It is generally feasible to dissolve the crude salt in a solvent in a temperature range from a room temperature (25°C) to about a boiling point of the solvent used, and then, causing the crystalline precipitate at -40 to 80°C. In the case of using water as the solvent, for example, the perfluoroalkanesulfinate salt can be obtained with high purity by dissolving or suspending the crude crystalline product in the solvent at a temperature of 25 to 100°C and causing the crystalline precipitate at a temperature of 10°C or lower.

There is no particular limitation on the reaction time in the fourth step. The optimal reaction time varies depending on the temperature, the amount of the substrate used and the like. As the starting raw material gets dissolved or suspended with the progress of the reaction, it is preferable to determine, as the end of the reaction, the time at which the precipitation of the salt has almost finished while monitoring the progress of the reaction.

There is also no particular limitation on the reactor used in the fourth step. The reactor can be selected from those made of metal materials, such as stainless steel, Hastelloy and Monel, with or without an inner lining of tetrafluoroethylene resin, chlorotrifluoroethylene resin, vinylidene fluoride resin, PFA resin, polypropylene resin, polyethylene resin, or glass etc.

In the fourth step, there can be used an organic solvent other than the water. Examples of the organic solvent are water-soluble solvents such as ether solvents including diethyl ether, dioxane, tetrahydrofuran and ethylene glycol dimethyl ether and ketone solvents including acetone, methyl ethyl ketone and methyl isobutyl ketone. The organic solvent may be used in combination with water. The amount of the solvent used relative to the perfluoroalkanesulfinate salt is set as appropriate in the range of generally 0.5 to 10 times in volume, preferably 1 to 7 times in volume. There is however less merit in using the organic solvent other than water as the reaction proceeds sufficiently with the use of water.

A preferred embodiment of the post-treatment of the reaction solution after the fourth step will be explained below. It has been found that it is possible in the fourth step to recover the impurity remaining as the reaction by-product including the above-mentioned compound and mix the recovered impurity in the reaction solution in the post-treatment operation after the fourth step. In other words, it is feasible that the impurity including the alkali metal halide can be recovered from the separated solid and mixed into the reaction solution before the purification of the fourth step.

Herein, the reaction solution before the purification refers to the reaction solution immediately after the addition of at least one compound selected from the group consisting of alkali metal halides, alkaline-earth metal halides, alkali metal hydroxides, alkaline-earth metal hydroxides, alkali metal sulfates and alkaline-earth metal sulfates in the fourth step.

This operation is one preferred embodiment for substantial reduction in wastes. (See the following Scheme 10.)

[Chem. 18]

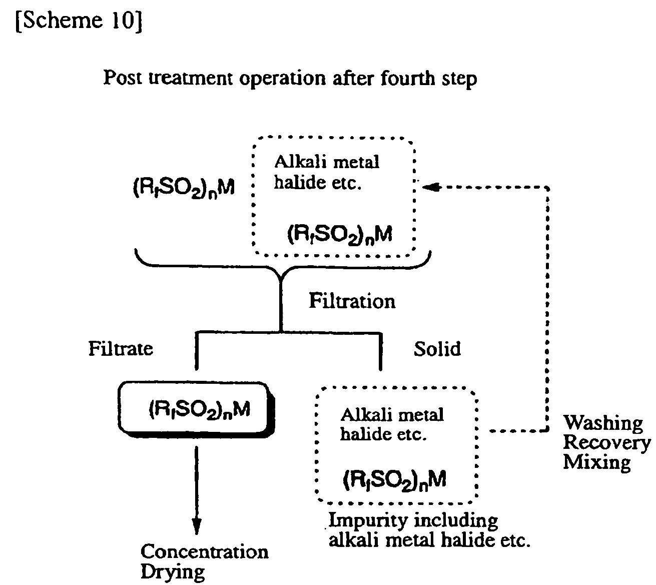

As mentioned above, the perfluoroalkanesulfinate salt and the impurity including the alkali metal halide or alkaline-earth metal halide are contained in the separated solid after the purification of the fourth step. It is feasible to selectively dissolve and recover the perfluoroalkanesulfinate salt by washing the separated solid with an aqueous solution of an alkali metal halide or alkaline-earth metal halide. This solution can be mixed in the reaction solution before the purification of the fourth step so as to thereby allow substantial reduction in the process loss of the above operation.

In this way, it is possible to produce the perfluoroalkanesulfinate salt with high purity through the above-mentioned first to fourth steps.

In the present invention, the perfluoroalkanesulfinate salt produced through the above first to fourth steps can be converted to a perfluoroalkanesulfinic acid (CF₃SO₂H) by acid decomposition.

The acid decomposition will be next explained below. Herein, the "acid decomposition" refers to a decomposition reaction under the action of an acid.

There is no particular limitation on the acid used as long as the acid is a Brönsted acid. Examples of the acid are: inorganic acids such as hydrochloric acid, sulfuric acid, nitric acid, phosphoric acid, silicic acid, hydrobromic acid and boric acid; and organic acids such as formic acid, acetic acid, propionic acid, butyric acid, valeric acid, pivalic acid, oxalic acid, succinic acid, adipic acid, crotonic acid, methanesulfonic acid and trifluoromethanesulfonic acid. The amount of the acid used varies depending on the valency of the acid. In the case of the monovalent acid, the amount of the acid used is 1 mol or more, preferably 1 to 5 mol, per 1 mol of the perfluoroalkanesulfinate salt. In the case of the divalent acid, the amount of the acid used is 0.5 mol or more, preferably 0.5 to 2.5 mol, per 1 mol of the perfluoroalkanesulfinate salt.

The acid decomposition can be carried out under the coexistence of water. There is no particular limitation on the amount of the water used as long as the water is used in an amount of 1 mol or more per 1 mol of the perfluoroalkanesulfinate salt substrate material. The amount of the water is preferably 1 to 1000 mol, more preferably 1 to 100 mol, per 1 mol of the perfluoroalkanesulfinate salt. In the case where the above-mentioned acid contains water, this water can be used. It is feasible, but not preferable, to use additional water in view of the decrease in the yield per volume of the reactor and the need to equip with the larger reactor. There is no particular limitation on the concentration of the acid. Preferably, the concentration of the acid is 10 to 90%.

The reaction temperature is generally -30 to 100°C, preferably -10 to 100°C, more preferably 0 to 100°C. When the reaction temperature is lower than -30°C, the reaction speed becomes lowered. When the reaction temperature is higher than 100°C, there may occur vaporization of the perfluorosulfinic acid generated gradually in the reaction system and water used in the acid decomposition.

In the case of performing the reaction at ordinary temperatures and pressures, there is no particular limitation on the reactor used in the acid decomposition as long as the reactor is capable of resisting and withstanding the pressure. As the reactor, there can be used the one having an inner lining of tetrafluoroethylene resin, chlorotrifluoroethylene resin, vinylidene fluoride resin, PFA resin, polypropylene resin, polyethylene resin, or glass etc. Further, a glass container can be used as the reactor. The reaction proceeds even in a reactor having an inner wall of stainless steel, iron or the like. There is however a possibility that such a metal reactor becomes corroded by the perfluoroalkanesulfinic acid. It is thus preferable to use the above-mentioned type of reactor.

There is also no particular limitation on the post-treatment operation after the reaction. As a by-product such as inorganic acid salt appears with the progress of the reaction, the perfluoroalkanesulfinic acid can be obtained by subjecting the reaction solution to filtration and then to any ordinary treatment means such as distillation.

The present invention will be described in more detail below by way of the following examples. It should be noted that these examples are illustrative and are not intended to limit the present invention thereto. Herein, "%" of the composition analysis values represent "wt%" of composition obtained by directly measuring the reaction mixtures by ion chromatography.

### Example 1

### [First Step]

### Preparation of Perfluoroalkanesulfinic Acid Hydrazinium salt

A jacketed packed tower of 30 mm diameter in section, in which 6-mm × 6-mm stainless steel (SUS) raschig ring had been packed to a height of 0.54 mm, was placed in a 500-ml reservoir vessel. The reservoir vessel was then charged with 214 g of a 30wt% aqueous hydrazine solution. The aqueous hydrazine solution was circulated through the packed tower by a pump and heated to about 57°C. When the temperature of the aqueous hydrazine solution was stabilized, a mixed gas generated from an electrolytic cell and containing trifluoromethanesulfonyl fluoride (hydrogen:trifluoromethanesulfonyl fluoride = 4:1) was introduced into the packed tower under the condition of 170 mol/hr·m² so that the trifluoromethanesulfonyl fluoride was absorbed in and reacted with the hydrazine solution. After introducing 63 g of trifluoromethanesulfonyl fluoride to the packed tower, 96 g of a 48% aqueous potassium hydroxide solution was once added to the reservoir vessel. Further, 38 g of trifluoromethanesulfonyl fluoride was introduced. The reaction solution was pump-circulated for 1 hour while introducing nitrogen. The resulting reaction solution was taken out and analyzed for its gas absorption amount by ¹⁹F NMR. It was confirmed from the analytical results that 95% of the trifluoromethanesulfonyl fluoride was converted to trifluoromethanesulfinic acid hydrazinium salt. The reaction solution was subjected to the following second step without purification.

### [Second Step]

### Formation of Perfluoroalkanesulfinic Acid Alkali Metal Salt

To the absorption solution obtained in the first step, 60.9 g of 48% potassium hydroxide was added whereby the hydrazinium salt was converted to potassium trifluoromethanesulfinate. The potassium trifluoromethanesulfinate was subjected to the third step without purification.

### [Third Step]

### Precipitation of Perfluoroalkanesulfinic Acid Alkali Metal Salt

To the potassium trifluoromethanesulfinate obtained in the second step, 131 g of potassium fluoride was added. The resulting mixture was subjected to distillation under reduced pressure at 60°C, thereby distillating 99.7 g of an aqueous hydrazine solution.
As 13.8 g of hydrazine was contained in this distillate solution, the recovery rate of the hydrazine was 41 %. The absorption solution after the distillation was again mixed with 100 g of water and subjected to distillation in the same manner as above, thereby distillating 137.7 g of an aqueous hydrazine solution. In the second distillate solution, 12.3 g of hydrazine was contained. Thus, in total, 78% of the hydrazine was recovered by the above concentration operation. The resulting concentrated solution was cooled to about 35°C whereby potassium trifluoromethanesulfinate (CF₃SO₂K) was precipitated out of the solution. The precipitate was filtered out by a Nutsche filter. As a result, 142.7 g of a crude crystalline product of potassium trifluoromethanesulfinate was yielded. In this crude crystalline product, 26.3 g of potassium fluoride, 96.7 g of potassium trifluoromethanesulfinate and 19.1 g of water were contained.

### [Fourth Step]

### Purification of Perfluoroalkanesulfinic Acid Alkali Metal Salt

To the crude crystalline product obtained in the third step, 280 g of water and 39.4 g of sodium sulfate (Na₂SO₄) were added. The resulting solution was heated to 60°C and stirred for 1 hour at 60°C, followed by distillating 245 g of water under reduced pressure. After the distillation, the reaction system was cooled until the inside temperature of the reaction system reached 10°C or lower. The thus-precipitated crystalline solid was filtered out by a Nutsche filter. The resulting filtrate was concentrated and dried/hardened, thereby yielding 90.8 g of a white crystalline solid product of potassium trifluoromethanesulfinate. This crystalline solid product was analyzed by ion chromatography. It was confirmed from the analytical results that the purity and yield of the purified product were 99.1 wt% and 86.5%, respectively.

In this step, 75.3 g of the solid was also obtained by the above filtration operation. In the solid, 23.9 g (0.57 mol) of sodium fluoride, 7.4 g (0.04 mol) of potassium trifluoromethanesulfinate, 40.8 g (0.23 mol) of potassium sulfate and 3.1 g of water were contained. On the Nutsche filter, this crystalline solid was washed with 45.2 g of a 35% aqueous potassium fluoride solution. Consequently, 7.4 g (0.04 mol) of potassium triluforomethanesulfinate, 14.3 g (0.24 mol) of potassium fluoride and 30.7 g of water were contained in the wash solution. When the wash solution was added to the reaction solution before the addition of sodium sulfate in the fourth step, the yield of the fourth step could be improved by about 7%.

The results of comparison of the amounts of wastes generated during the production of 1 kg of potassium trifluoromethanesulfinate in Example 1 and the amounts of wastes generated during the production of the same potassium sulfinate by the process of Patent Document 3 are indicated in the following TABLE.

**TABLE 1**

| Waste amounts (kg/kg) in production of 1 kg of CF₃SO₂K | | |
|---|---|---|
| | Example 1 | Patent Document 3 |
| Solid waste (sulfate etc.) | 1.7 | 6.0 |
| Waste organic solvent | 0 | 10.0 |
| Waste water | 1.3 | 8.7 |

As is apparent from TABLE 1, it was possible in the present invention, through the above first to fourth steps, to recover and use the by-product as the reactant in the subsequent reaction step and substantially reduce the waste amounts as compared to Patent Document 3.

### Example 2

The first to third steps were performed in the same manner as in Example 1, thereby yielding 23.1 g of a crude crystalline product of trifluoromethanesulfinate salt. In this crude crystalline product, 3.5 g of potassium fluoride, 16.4 g of potassium trifluoromethanesulfinate and 3.1 g of water were contained. The crude crystalline product was subsequently subjected to the fourth step as follows.

### [Fourth Step]

### Purification of Perfluoroalkanesulfinic Acid Alkali Metal Salt

To the crude crystalline product obtained in the third step, 36.9 g of water and 3.5 g of sodium chloride were added. The resulting mixture was heated to 60°C and stirred for 1 hour at 60°C, followed by distillating 30 g of water under reduced pressure. After the distillation, the reaction system was cooled until the inside temperature of the reaction system reached 10°C or lower. The thus-precipitated crystalline solid was filtered out by a Nutsche filter. The resulting filtrate was concentrated and dried/hardened, thereby yielding 16.0 g of a white crystalline solid product of potassium trifluoromethanesulfinate. This crystalline solid product was analyzed by ion chromatography. It was confirmed from the analytical results that the purity and yield of the purified product were 96.0 wt% and 89.3%, respectively.

### Example 3

The first to third steps were performed in the same manner as in Example 1, thereby yielding 17.3 g of a crude crystalline product of trifluoromethanesulfinate salt. In this crude crystalline product, 2.6 g of potassium fluoride, 12.3 g of potassium trifluoromethanesulfinate and 2.3 g of water were contained.

Subsequently, the crude crystalline product was subjected to the fourth step as follows.

### [Fourth Step]

### Purification of Perfluoroalkanesulfinic Acid Alkali Metal Salt

To the crude crystalline product obtained in the third step, 27.7 g of water and 1.8 g of sodium chloride were added. The resulting mixture was sufficiently stirred, followed by adding thereto 5.74 g of 5M sulfuric acid, stirring the mixture, and then, distillating 22.3 g of water under reduced pressure. After the distillation, the reaction system was cooled until the inside temperature of the reaction system reached 10°C or lower. The thus-precipitated crystalline solid was filtered out by a Nutsche filter. The resulting filtrate was concentrated and dried/hardened, thereby yielding 11.2 g of a white crystalline solid product of potassium trifluoromethanesulfinate. This crystalline solid product was analyzed by ion chromatography. It was confirmed from the analytical results that the purity and yield of the purified product were 98.7 wt% and 90.0%, respectively.

### Example 4

The first to third steps were performed in the same manner as in Example 1, except for using 80.8 g of perfluoroethanesulfinic acid fluoride, thereby yielding 113.3 g of a crude crystalline product of perfluoroethanesulfinate salt. In this crude crystalline product, 20.3 g of potassium fluoride, 75.5 g of potassium perfluoroethanesulfinate and 17.1 g of water were contained.

Subsequently, the crude crystalline product was subjected to the fourth step as follows.

### [Fourth Step]

### Purification of Perfluoroalkanesulfinic Acid Alkali Metal Salt

To the crude crystalline product obtained in the third step, 300 g of water and 48.0 g of sodium sulfate (Na₂SO₄) were added. The resulting mixture was heated to 60°C and stirred for 1 hour at 60°C, followed by distillating 165 g of water under reduced pressure. After the distillation, the reaction system was cooled until the inside temperature of the reaction system reached 10°C or lower. The thus-precipitated crystalline solid was filtered out by a Nutsche filter. The resulting filtrate was concentrated and dried/hardened, thereby yielding 68.3 g of a white crystalline solid product of potassium trifluoromethanesulfinate. This crystalline solid product was analyzed by ion chromatography. It was confirmed from the analytical results that the purity and yield of the purified product were 98.1 wt% and 76.9%, respectively.

### [Reference Example 1]

A jacketed packed tower of 30 mm diameter in section, in which 6-mm × 6-mm stainless steel (SUS) raschig ring had been packed to a height of 0.54 mm, was placed in a 500-ml reservoir vessel. The reservoir vessel was then charged with 515.5 g of a 20.5wt% aqueous hydrazine solution. The aqueous hydrazine solution was circulated through the packed tower by a pump and heated to about 47°C. When the temperature of the aqueous hydrazine solution was stabilized, trifluoromethanesulfonyl fluoride vaporized by a flowmeter was introduced into the packed tower under the condition of 170 mol/hr·m² so that the trifluoromethanesulfonyl fluoride was absorbed in and reacted with the hydrazine solution. After introducing 100 g (0.658 mol) of trifluoromethanesulfonyl fluoride, the reaction solution was pump-circulated for 1 hour while introducing nitrogen. The resulting reaction solution was taken out. To the reaction solution, 321 g (3.28 mol) of concentrated sulfuric acid was added with cooling. The thus-precipitated solid was subjected to suction filtration. The resulting filtrate was subjected to simple distillation under reduced pressure of 30 hPa, thereby obtaining a main distillate at a boiling point of 72.1 to 73.7°C. To the main distillate, 70.9 g (0.607 mol) of a 48% aqueous potassium hydroxide solution was added. The thus-formed mixture was subjected to water distillation and dried, thereby yielding 86.7 g of a white solid product of potassium trifluoromethanesulfinate with a purity of 98.0% (determined by ¹⁹F NMR) and a yield of 76.5%.

In this reference example, the potassium trifluoromethanesulfinate was obtained by reaction of the reaction solution obtained after the first step with concentrated sulfuric acid and potassium hydroxide. However, the distillation operation made it difficult to control the reaction. Further, the yield of the reaction was deteriorated to some extent due to decomposition of part of the sulfinic acid. It was thus somewhat unsuitable for industrial production.

### [Reference Example 2]

The first step was performed in the same manner as in Example 1. The reaction mixture containing perfluoroalkanesulfinic acid alkali metal salt after the second step was subjected to distillation under reduced pressure, thereby recovering hydrazine. As a result, 136 g of an aqueous hydrazine solution was recovered. As 5.8 g of hydrazine was contained in this distillate solution, the recovery rate of the hydrazine was 17%. (In this example, the subsequent third and fourth steps were not performed.)

It has been thus shown that, as compared to Example 1, the recovery rate of the hydrazine was lowered when the hydrazine recovery operation was performed before the third step i.e. after the second step.

### Example 5

Into a 300-ml three-neck flask equipped with a manual reflux condenser and a packed tower in which 6-mm diameter PFA raschig ring had been packed to 13 cm, 100 g (0.581 mol) of potassium trifluoromethanesulfinate produced by the process of Example 1 and 114 g (1.16 mol) of sulfuric acid were charged. The resulting mixture was stirred for 30 minutes at room temperature and subsequently subjected to distillation under reduced pressure by setting the temperature of the jacket to 85°C and the degree of vacuum to 30 hPa. In this distillation, a fraction distilled at a vacuum degree of 14 to 20 hPa and a distillation temperature of 58 to 66°C was extracted as a main distillate. As a result, the target trifluoromethanesulfinic acid was obtained with a purity of 93.4% (analyzed by ion chromatography) and a yield of 41 g (62.3%).

## Claims

1. A process for producing a perfluoroalkanesulfinate salt of the formula [1] ]
[Chem. 19]
**(R_{f}SO₂)ₙM** [1]
where R_{f} represents a saturated or unsaturated C₁-C₄ straight or branched perfluoroalkyl group; M represents an alkali metal or an alkaline-earth metal; and n represents an integer that is equal to the valency of the metal,
the process comprising the following steps:
a first step of reacting a perfluoroalkanesulfonyl halide of the formula [2] with hydrazine to prepare a perfluoroalkanesulfinic acid hydrazinium salt (R_{f}SO₂H·N₂H₄)
[Chem. 20]
**R_{f}SO₂X** [2]
where R_{f} is the same as defined in the formula [1]; X represents fluorine, chlorine, bromine or iodine;
a second step of forming a perfluoroalkanesulfinate salt by reaction of the perfluoroalkanesulfinic acid hydrazinium salt prepared in the first step with at least one base selected from the group consisting of alkali metal hydroxides, alkali metal carbonates, alkali metal hydrogencarbonates, alkaline-earth metal carbonates and alkaline-earth metal hydrogencarbonates;
a third step of adding an alkali metal halide or alkaline-earth metal halide, in which the alkali metal or alkaline-earth metal is the same as that used in the second step, to the perfluoroalkanesulfinate salt formed in the second step and thereby obtaining the perfluoroalkanesulfinate salt as a crystalline precipitate; and
a fourth step of purifying the perfluoroalkanesulfinate salt obtained in the third step with the addition of at least one compound selected from the group consisting of alkali metal halides, alkaline-earth metal halides, alkali metal hydroxides, alkaline-earth metal hydroxides, alkali metal sulfates and alkaline-earth metal sulfates.

2. A process according to claim 1 for producing a potassium perfluoroalkanesulfinate of the formula [3]
[Chem. 21]
**R_{f}SO₂K** [3]
where R_{f} represents a saturated or unsaturated C₁-C₄ straight or branched perfluoroalkyl group
the process comprising the following steps:
a first step of reacting a perfluoroalkanesulfonyl fluoride of the formula [4] with hydrazine to prepare a perfluoroalkanesulfinic acid hydrazinium salt (R_{f}SO₂H·N₂H₄)
[Chem. 22]
**R_{f}SO₂F** [4]
where R_{f} is the same as defined in the formula [3];
a second step of forming a potassium perfluoroalkanesulfinate by reaction of the perfluoroalkanesulfinic acid hydrazinium salt prepared in the first step with either potassium hydroxide, potassium carbonate or potassium hydrogencarbonate;
a third step of adding potassium fluoride to the potassium perfluoroalkanesulfinate formed in the second step and thereby obtaining the potassium perfluoroalkanesulfinate as a crystalline precipitate; and
a fourth step of purifying the potassium perfluoroalkanesulfinate obtained in the third step with the addition of at least one compound selected from the group consisting of lithium chloride, sodium chloride, magnesium chloride, calcium chloride, lithium sulfate, sodium sulfate, magnesium sulfate, calcium sulfate and barium sulfate.

3. The process according to claim 1, wherein the first step is performed by charging the hydrazine in a reactor, and then, adding with stirring the perfluoroalkanesulfonyl halide at one time, sequentially or continuously, to the hydrazine in the reactor.

4. The process according to any one of claims 1 to 3, wherein the first step is performed at a reaction temperature of -10 to 110°C.

5. The process according to any one of claims 1 to 4, wherein the perfluoroalkanesulfinic acid hydrazinium salt prepared in the first step is used in the second step without purification.

6. The process according to claim 1 or 2, wherein, in the second step, the perfluoroalkanesulfinic acid hydrazinium salt is reacted with an equivalent molar amount of the base.

7. The process according to claim 1 or 2, wherein the perfluoroalkanesulfinate salt formed in the second step is used in the third step without purification.

8. The process according to claim 1 or 2, wherein the hydrazine present as a by-product in the third step is recovered and used as a reactant in the first step.

9. The process according to claim 1, wherein the alkali metal halide or alkaline-earth metal halide present in the reaction system is recovered and used as an additive in the third step.

10. The process according to claim 1, wherein an impurity present as a by-product including the at least one base selected from the group consisting of alkali metal hydroxides, alkali metal carbonates, alkali metal hydrogencarbonates, alkaline-earth metal hydroxides, alkaline-earth metal carbonates and alkaline-earth metal hydrogencarbonates is recovered and mixed in a reaction solution before the purification of the fourth step.

11. A process for producing a perfluoroalkanesulfinic acid, comprising the steps of preparing a perfluoroalkanesulfinate salt by the process according to any one of claims 1 to 10 and then causing acid decomposition of said perfluoroalkanesulfinate salt.

## Patentansprüche

1. Verfahren zum Herstellen eines Perfluoralkansulfinatsalzes gemäß der Formel [1]
[Chem. 19]
(R_{f}SO₂)ₙM [1]
worin R_{f} eine gesättigte oder ungesättigte, geradkettige oder verzweigte C₁-C₄-Perfluoralkylgruppe ist, M ein Alkalimetall oder ein Erdalkalimetall bedeutet und n eine ganze Zahl ist, welche der Valenz des Metalls entspricht,
wobei das Verfahren die nachfolgenden Schritte umfasst:
einen ersten Schritt des Reagierens eines Perfluoralkansulfonylhalogenids gemäß der Formel [2] mit Hydrazin, um ein Perfluoralkansulfinsäurehydrazinsalz (R_{f}SO₂H·N₂H₄) herzustellen,
[Chem. 20]
R_{f}SO₂X [2]
worin R_{f} derselbe wie der zuvor in der Formel [1] definierte Rest ist, X Fluor, Chlor, Brom oder Iod ist,
einen zweiten Schritt des Ausbildens eines Perfluoralkansulfinatsalzes durch Reaktion des in dem ersten Schritt hergestellten Perfluoralkansulfinsäurehydrazinsalzes mit wenigstens einer Base ausgewählt aus der Gruppe bestehend aus Alkalimetallhydroxiden, Alkalimetallcarbonaten, Alkalimetallhydrogencarbonaten, Erdalkalimetallcarbonaten und Erdalkalimetallhydrogencarbonaten,
einen dritten Schritt der Zugabe eines Alkalimetallhalogenids oder eines Erdalkalimetallhalogenids, bei dem das Alkalimetall oder Erdalkalimetall dasselbe ist, welches in dem zweiten Schritt eingesetzt wird, zu dem Perfluoralkansulfinatsalz, welches in dem zweiten Schritt gebildet worden ist, und dadurch Erhalten eines Perfluoralkansulfinatsalzes als ein kristallines Präzipitat und
einen vierten Schritt des Reinigens des in dem dritten Schritt erhaltenen Perfluoralkansulfinatsalzes durch die Zugabe wenigstens einer Verbindung ausgewählt aus der Gruppe bestehend aus Alkalimetallhalogeniden, Erdalkalimetallhalogeniden, Alkalimetallhydroxiden, Erdalkalimetallhydroxiden, Alkalimetallsulfaten und Erdalkalimetallsulfaten.

2. Verfahren nach Anspruch 1 zum Herstellen eines Kaliumperfluoralkansulfinats gemäß der Formel [3]
[Chem.21]
R_{f}SO₂K [3]
worin R_{f} eine gesättigte oder ungesättigte, geradkettige oder verzweigtkettige C₁-C₄-Perfluoralkylgruppe ist,
wobei das Verfahren die nachfolgenden Schritte umfasst:
einen ersten Schritt des Reagierens von Perfluoralkansulfonylfluorid gemäß der Formel [4] mit Hydrazin, um einen Perfluoralkansulfinsäurehydrazinsalz (R_{f}SO₂H·N₂H₄) herzustellen
[Chem. 22]
R_{f}SO₂F [4]
worin R_{f} derselbe wie der zuvor in der Formel [3] definierte Rest ist,
einen zweiten Schritt des Ausbildens eines Kaliumperfluoralkansulfinats durch Reaktion des in dem ersten Schritt hergestellten Perfluoralkansulfinsäurehydrazinsalzes mit entweder Kaliumhydroxid, Kaliumcarbonat oder Kaliumhydrogencarbonat,
einen dritten Schritt der Zugabe von Kaliumfluorid zu dem Kaliumperfluoralkansulfinat, welches in dem zweiten Schritt gebildet worden ist, und dadurch Erhalten des Kaliumperfluoralkansulfinats als ein kristallines Präzipitat und
einen vierten Schritt des Reinigens des in dem dritten Schritt erhaltenen Kaliumperfluoralkansulfinats durch die Zugabe wenigstens einer Verbindung ausgewählt aus der Gruppe bestehend aus Lithiumchlorid, Natriumchlorid, Magnesiumchlorid, Calciumchlorid, Lithiumsulfat, Natriumsulfat, Magnesiumsulfat, Calciumsulfat und Bariumsulfat.

3. Verfahren nach Anspruch 1, wobei der erste Schritt durch Zuführen von Hydrazin in einen Reaktor und dann Zugabe unter Rühren des Perfluoralkansulfonylhalogenids zu einer Zeit, sequentiell oder kontinuierlich, zu dem Hydrazin in dem Reaktor durchgeführt wird.

4. Verfahren nach einem der Ansprüche 1 bis 3, wobei der erste Schritt bei einer Reaktionstemperatur von -10 bis 110°C durchgeführt wird.

5. Verfahren nach einem der Ansprüche 1 bis 4, wobei das in dem ersten Schritt hergestellte Perfluoralkansulfinsäurehydrazinsalz in dem zweiten Schritt ohne Reinigung eingesetzt wird.

6. Verfahren nach Anspruch 1 oder 2, wobei in dem zweiten Schritt das Perfluoralkansulfinsäurehydrazinsalz mit einer äquivalenten molaren Menge der Base reagiert wird.

7. Verfahren nach Anspruch 1 oder 2, wobei das in dem zweiten Schritt gebildete Perfluoralkansulfinatsalz in dem dritten Schritt ohne Reinigung eingesetzt wird.

8. Verfahren nach Anspruch 1 oder 2, wobei das als ein Nebenprodukt vorliegende Hydrazinsalz in dem dritten Schritt wiedergewonnen wird und in dem ersten Schritt als ein Reaktand eingesetzt wird.

9. Verfahren nach Anspruch 1, wobei das in dem Reaktionssystem vorliegende Alkalimetallhalogenid oder Erdalkalimetallhalogenid wiedergewonnen wird und in dem dritten Schritt als ein Additiv eingesetzt wird.

10. Verfahren nach Anspruch 1, wobei ein als Verunreinigung vorliegendes Nebenprodukt enthaltend die wenigstens eine Base ausgewählt aus der Gruppe bestehend aus Alkalimetallhydroxiden, Alkalimetallcarbonaten, Alkalimetallhydrogencarbonaten, Erdalkalimetallhydroxiden, Erdalkalimetallcarbonaten und Erdalkalimetallhydrogencarbonaten wiedergewonnen und vor der Reinigung des vierten Schritts in einer Reaktionslösung vermischt wird.

11. Verfahren zum Herstellen einer Perfluoralkansulimsäure umfassend die Schritte des Herstellens eines Perfluoralkansulfinatsalzes durch ein Verfahren nach einem der Ansprüche 1 bis 10 und dann Verursachen einer Zersetzung des Perfluoralkansulfinatsalzes.

## Revendications

1. Procédé pour produire un sel perfluoralcanesulfinate de formule [1]
[Chem. 19]
(R_{f}SO₂)ₙM [1]
dans laquelle R_{f} représente un groupe perfluoralkyle droit ou ramifié en C₁ à C₄ saturé ou insaturé ; M représente un métal alcalin ou un métal alcalinoterreux ; et n représente un nombre entier qui est égal à la valence du métal,
le procédé comprenant les étapes suivantes :
une première étape de réaction d'un halogénure de perfluoralcanesulfonyle de formule [2] avec de l'hydrazine pour préparer un sel d'hydrazinium d'acide perfluoralcanesulfinique (R_{f}SO₂H·N₂H₄)
[Chem 20]
RfSO₂X [2]
dans lequel R_{f} répond à la définition indiquée pour la formule [1] ; X représente un atome de fluor, de chlore, de brome ou d'iode ;
une deuxième étape de formation d'un sel perfluoralcanesulfinate par réaction du sel d'hydrazinium d'acide perfluoralcanesulfinique préparé dans la première étape avec au moins une base choisie dans le groupe consistant en des hydroxydes de métaux alcalins, des carbonates de métaux alcalins, des carbonates acides de métaux alcalins, des carbonates de métaux alcalinoterreux et des carbonates acides de métaux alcalinoterreux ;
une troisième d'addition d'un halogénure de métal alcalin ou d'un halogénure de métal alcalinoterreux, dans laquelle le métal alcalin ou le métal alcalinoterreux est identique à celui utilisé dans la deuxième étape, au sel perfluroalcanesulfinate formé dans la deuxième étape et ainsi d'obtention du sel perfluoralcanesulfinate sous forme d'un précipité cristallin ; et
une quatrième étape de purification du sel perfluoralcanesulfinate obtenu dans la troisième étape par addition d'au moins un composé choisi dans le groupe consistant en des halogénures de métaux alcalins, des halogénures de métaux alcalinoterreux, des hydroxydes de métaux alcalins, des hydroxydes de métaux alcalinoterreux, des sulfates de métaux alcalins et des sulfates de métaux alcalinoterreux.

2. Procédé suivant la revendication 1, pour produire un perfluoralcanesulfinate de potassium de formule [3]
[Chem. 21]
R_{f}SO₂K [3]
dans laquelle R_{f} représente un groupe perfluoralkyle droit ou ramifié en C₁ à C₄ saturé ou insaturé
le procédé comprenant les étapes suivantes :
une première étape de réaction d'un fluorure de perfluoralcanesulfonyle de formule [4] avec de l'hydrazine pour préparer un sel d'hydrazinium d'acide perfluoralcanesulfinique (R_{f}SO₂H.N₂H₄)
[Chem. 22]
R_{f}SO₂F [4]
dans lequel R_{f} répond à la définition indiquée pour la formule [3] ;
une deuxième étape de formation d'un perfluoralcanesulfinate de potassium par réaction du sel d'hydrazinium d'acide perfluoralcanesulfinique préparé dans la première étape avec de l'hydroxyde de potassium, du carbonate de potassium ou du carbonate acide de potassium ;
une troisième étape d'addition de fluorure de potassium au perfluoralcanesulfinate de potassium formé dans la deuxième étape et ainsi d'obtention du perfluoralcanesulfinate de potassium sous forme d'un précipité cristallin ; et
une quatrième étape de purification du perfluoralcanesulfinate de potassium obtenu dans la troisième étape par addition d'au moins un composé choisi dans le groupe consistant en le chlorure de lithium, le chlorure de sodium, le chlorure de magnésium, le chlorure de calcium, le sulfate de lithium, le sulfate de sodium, le sulfate de magnésium, le sulfate de calcium et le sulfate de baryum.

3. Procédé suivant la revendication 1, dans lequel la première étape est mise en oeuvre en introduisant l'hydrazine dans un réacteur, puis en ajoutant sous agitation l'halogénure de perfluoralcanesulfonyle en une fois, de manière séquentielle ou continue, à l'hydrazine dans le réacteur.

4. Procédé suivant l'une quelconque des revendications 1 à 3, dans lequel la première étape est mise en oeuvre à une température réactionnelle de -10 à 110°C.

5. Procédé suivant l'une quelconque des revendications 1 à 4, dans lequel le sel d'hydrazinium d'acide perfluoralcanesulfinique préparé dans la première étape est utilisé dans la deuxième étape sans purification.

6. Procédé suivant la revendication 1 ou 2, dans lequel, dans la deuxième étape, le sel d'hydrazinium d'acide perfluoralcanesulfinique est amené à réagir avec une quantité molaire équivalente de la base.

7. Procédé suivant la revendication 1 ou 2, dans lequel le sel perfluoralcanesulfinate formé dans la deuxième étape est utilisé dans la troisième étape sans purification.

8. Procédé suivant la revendication 1 ou 2, dans lequel l'hydrazine présente comme sous-produit dans la troisième étape est récupérée et utilisée comme corps réactionnel dans la première étape.

9. Procédé suivant la revendication 1, dans lequel l'halogénure de métal alcalin ou l'halogénure de métal alcalinoterreux présent dans le système réactionnel est récupéré et utilisé comme additif dans la troisième étape.

10. Procédé suivant la revendication 1, dans lequel une impureté présente comme sous-produit comprenant ladite au moins une base choisie dans le groupe consistant en des hydroxydes de métaux alcalins, des carbonates de métaux alcalins, des carbonates acides de métaux alcalins, des hydroxydes de métaux alcalinoterreux, des carbonates de métaux alcalinoterreux et des carbonates acides de métaux alcalinoterreux est récupérée et mélangée à une solution réactionnelle avant la purification dans la quatrième étape.

11. Procédé pour produire un acide perfluoralcanesulfinique, comprenant les étapes consistant à préparer un sel perfluoralcanesulfinate par le procédé suivant l'une quelconque des revendications 1 à 10 et ensuite à provoquer la décomposition par un acide dudit sel perfluoralcanesulfinate.
